(19) Europäisches Patentamt

European Patent Office

Office européen des brevets

(11) EP 1 279 960 A1

(12) EUROPEAN PATENT APPLICATION
published in accordance with Art. 158(3) EPC

(43) Date of publication:
29.01.2003 Bulletin 2003/05

(51) Int Cl.7: **G01N 33/552**, G01N 33/556, G01N 33/48, C12N 15/09

(21) Application number: 00962997.3

(22) Date of filing: 29.09.2000

(86) International application number:
PCT/JP00/06792

(87) International publication number:
WO 01/075447 (11.10.2001 Gazette 2001/41)

(84) Designated Contracting States:
AT BE CH CY DE DK ES FI FR GB GR IE IT LI LU MC NL PT SE
Designated Extension States:
AL LT LV MK RO SI

(30) Priority: 04.04.2000 JP 2000102723
09.08.2000 JP 2000240924

(71) Applicant: Toyo Kohan Co., Ltd.
Tokyo 102-8447 (JP)

(72) Inventors:
• TANGA, M.,
Toyo Kohan Co.,Ltd. Technical Res. Lab.
Kudamatsu-shi, Yamaguchi 744-8611 (JP)

• OKAYAMA,H.,
Toyo Kohan Co.,Ltd.Technical Res. Lab.
Kudamatsu-shi, Yamaguchi 744-8611 (JP)
• OKAMURA,H.,
Toyo Kohan Co.,Ltd.Technical Res. Lab
Kudamatsu-shi, Yamaguchi 744-8611 (JP)
• EHARA, K.,
Toyo Kohan Co.,Ltd.Technical Res. Lab.
Kudamatsu-shi, Yamaguchi 744-8611 (JP)
• TAKAGI, K.,
Toyo Kohan Co.,Ltd.Technical Res. Lab.
Kudamatsu-shi, Yamaguchi 744-8611 (JP)

(74) Representative: VOSSIUS & PARTNER
Siebertstrasse 4
81675 München (DE)

(54) **SLIDE GLASS HAVING SURFACE TREATMENT LAYER FORMED THEREON**

(57) A slide glass, characterized in that it has a surface treatment layer formed thereon, the layer preferably comprising diamond, a diamond-like carbon or the like, and is capable of carrying oligonucleotide probes or the like on the surface thereof; and a method for analyzing a gene, characterized as analyzing a gene through carrying the gene on the surface of the above slide glass having a surface treatment layer formed thereon. The method for analyzing a gene is almost free from the problem that a spot having an organism sample such as DNA or a protein attached thereto is often unclear and difficult to observe.

Fig. 1

DLC surface

Utilization of amino group of A, G, C

H₂N ▬▭▭▭▭▭

Amide bonding

## Description

Technical Field

The present invention relates to slide glasses used in analysis of biogenic materials such as genes or proteins which are utilized in genetic analysis, diagnosis, therapeutic treatment, and the like, and to a method for analyzing biogenic materials such as genes, proteins, peptides, and the like, using the slide glasses.

Background Art

As for slide glasses so far used for genetic analysis, etc., those made of glass tips, that is, those processed so that 10,000 or more of genes such as DNA (Deoxyribonucleic Acid) fragments (DNA probes) can be placed on their surface have widely been used.

Using such a tip as mentioned above, for example, when one intends to identify the base sequence of a DNA sample, tens of thousands of DNA fragments having already clarified and mutually different base sequences may be attached on the slide glass so that their respective positions can be followed; when a fluorescence-labeled DNA sample is poured thereon, the DNA fragment hybridizes with the probe having the complementary sequence among the DNA fragments (probes) attached on the slide glass. The hybridized portion can be identified as a spot on the slide glass by means of fluorometric measurement. Thus, the sequence of the DNA fragment in the sample can be elucidated.

The slide glasses for genetic analysis, since a certain DNA base sequence can readily be identified thereon in such a way, have been utilized in genetic analyses such as analyses of biogenic genomes, monitoring of gene expression, genomic mismatch, and the like, as well as in genetic diagnosis such as detection of mutation of oncogenes or development of drugs.

Utilizing the above-described slide glass for genetic analyses, a DNA sample can be amplified and the spot appearing on the glass by irradiation of fluorescence is analyzed for judgment.

In the so far used slide glass for genetic analyses, however, the glass had to be pretreated, e.g., washed, before analysis of the above-described spot, by which operation the spotted DNA fragment was washed away. In many cases, accordingly, the spots could not be detected clearly.

The purpose of the invention is to solve the problem that the fluorescent detection is unclear in the slide glasses used in the prior art for genetic analyses.

Disclosure of Invention

The slide glass of the invention is characterized in that a particular surface treatment layer is formed on the surface of the glass on which a biogenic material such as DNA probe, protein, peptide, etc., can be placed, by which layer the spotted DNA fragment, protein, peptide, and so on is firmly immobilized thereon without washed away during washing and shows a definite fluorescent spot by irradiation of fluorescence.

That is, the slide glass of the invention is characterized in that a surface treatment layer is formed on the surface of a glass substrate.

In such a slide glass, the above-described surface treatment layer is preferably diamond, diamond-like carbon, or carbon-type material, or a mixture thereof, or a laminating layer thereof.

In such a slide glass, the optical transmittance of the slide glass on which the above-described diamond-like carbon is formed is preferably 50% or more at a wavelength of 400 nm to 500 nm.

In such a slide glass, the optical transmittance of the slide glass on which the above-described diamond-like carbon is formed is preferably 70% or more at a wavelength of 600 nm to 700 nm.

In such a slide glass, the thickness of the above-described surface treatment layer is preferably in 1 nm to 1000 nm.

In such a slide glass, the above-described diamond-like carbon may preferably be prepared by an ionizing vapor deposition process in a mixed gas containing 1-99 % by volume of hydrogen gas and residual 99-1 % by volume of methane gas.

The slide glass of the invention is characterized in that a reflection layer is formed on the back of the glass substrate.

The slide glass of the invention is characterized in that an oligonucleotide fragment is kept on the above-described surface treatment layer.

The slide glass of the invention is characterized in that a protein or peptide is kept on the above-described surface treatment layer.

The slide glass of the invention can be used in a method for analyzing a biogenic material such as gene, protein, peptide, and the like using a slide glass.

Brief Description of a Drawing

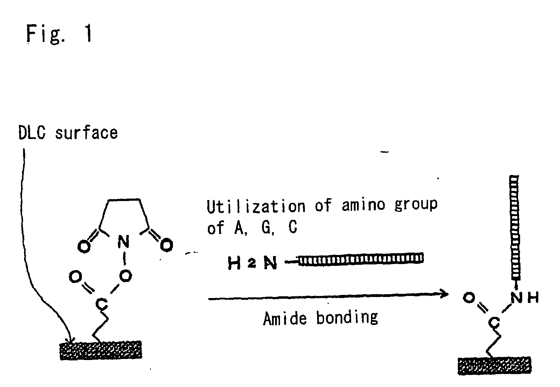

Fig. 1 is a general illustration of immobilization of a probe on the slide glass.

Best Mode for Carrying Out the Invention

In the slide glass of the invention, a surface treatment layer is formed on the surface of the glass, by which layer the affinity to a biogenic material such as gene, protein, peptide, and so on is enhanced; thus, it can preferably be used in a variety of analyses by placing a DNA sample on the surface treatment layer.

As such a surface treatment layer, it is preferable to use those coated with a carbon-type material such as diamond-like carbon (DLC), diamond, graphite, and the like.

The slide glass on which a surface treatment layer is formed with a carbon-type material such as diamond, diamond-like carbon (DLC), graphite, and the like, can firmly trap DNA probes.

That is, since carbon exhibits good chemical stability, it can resist the reaction in fixation of the DNA probe, etc. The reason is considered that when a probe such as DNA is fixed on the carbon, it can form a covalent bond with the carbon, as shown in Fig. 1, to firmly fix the DNA probe on the slide glass surface.

In addition, a mixture of the above-described carbon-type material such as diamond-like carbon (DLC), diamond, graphite, etc., with another material, for example, metal or ceramic, may be used as a surface treatment layer.

Alternatively, a laminating material consisting of the above-described carbon-type material such as diamond-like carbon (DLC), diamond, graphite, etc., and another material, may also be used as a surface treatment layer.

The immobilized probe, as shown in Fig. 1, stands nearly upright on the surface of the slide glass, and accordingly the immobilization density per unit area of the surface can be increased.

As the raw materials of diamond used in the above-described surface treatment layer, any of synthetic diamond, high pressure made diamond, or natural diamond may be used. There is no problem even though their structure is of single crystal or polycrystal. In view of the productivity, it is also preferable to form a diamond film by a gas-phase synthetic method, for example, a microwave plasma CVD (Chemical Vapor Deposit) process.

Alternatively, it is also possible to form an amorphous diamond-like carbon film as the surface treatment layer on the glass surface in view that it has equal performance to diamond and is economically advantageous. Diamond-like carbon consists of carbon atom similarly as in diamond or graphite and is called DLC because it has characteristics similar to diamond. The crystal structure of DLC is amorphous.

As for the above-described surface treatment layer, carbon-type materials such as graphite may also preferably be used. Diamond which has a regular tetrahedral structure consisting of a canter carbon and four corner carbons is an extremely hard cubic crystal forming the sp3 hybrid orbital.

As for the carbon-type materials, graphite or amorphous carbon is exemplified. Graphite is a hexagonal crystal having a structure in which sp2 hybrid hexagonal nets laminate in lamellar form through π-electrons. Amorphous carbon has no definite crystalline structure. Amorphous carbon also includes microcrystalline carbon of which the crystallinity is low.

In addition, there are carbon-type materials of intermediate structure including from amorphous carbon to graphite depending on the degree of the size or arrangement of the crystals. In these materials, there is no definite modification point. The carbon-type materials of the invention include graphite or amorphous carbon monomer, mixtures of graphite and amorphous carbon, and those having intermediate structure between amorphous carbon and graphite.

In the surface treatment layer of the invention, a mixture with the above-described carbon-type material such as diamond, DLC, graphite, and the like, may be used.

In addition, as the surface treatment layer of the invention, an alternately laminated layer of DLC film, diamond film, and carbon-type film may be used.

The thickness of the surface treatment layer of the invention, though not limited particularly, may be of 1 nm to 1000 nm. The thickness of less than 1 nm in the surface treatment layer gives no uniform layer due to too thin, and in some times non-coated portion is adversely produced on the basic glass substrate. On the other hand, coating over 1000 nm is not preferred because it readily causes separation of the layer due to stress in the surface treatment layer.

In view of the industrial productivity, the thickness of the surface treatment layer is preferably 10 nm to 500 nm, more preferably 30 to 200 nm.

The rate of formation of diamond in the surface treatment layer, when it is formed by the above-described plasma CVD process, may be properly determined according to the operational condition such as pressure in the reaction tank, bias voltage or substrate temperature.

The diamond concentration in the surface treatment layer may be controlled, for example, when the DLC film is formed by the plasma CVD process, by adjusting supply of $N_2$ gas as substituting gas to alter the H/C ratio in the hydrocarbon gas as a source.

When $N_2$ gas supply is increased, the uptake of $H_2$ in the DLC film is increased and as a result the concentration of diamond in DLC can be increased. On the other hand, when $N_2$ gas supply is decreased, the uptake of $H_2$ in the DLC film is decreased, and as a result the concentration of diamond in DLC can be decreased.

In the formation of the surface treatment layer of diamond, DLC, graphite, and so on to the glass substrate, a high-frequency plasma CVD process, an ionizing vapor deposition process, an arc vapor deposition process, a laser vapor deposition process, and the like can be applied.

In the high-frequency plasma CVD process, raw gas (methane) is decomposed by glow discharge generated between the electrodes by high frequency at 13.56 MHz, to synthesize a DLC film on the substrate. In the ionizing vapor deposition process, raw gas (benzene) is decomposed and ionized utilizing thermion generated by a tungsten filament to form a film on the substrate by bias voltage. The DLC film may also be formed in a mixed gas containing 1-99 % by volume of hydrogen gas and residual 99-1 % by volume of methane gas by the ionizing vapor deposition process.

The DLC film is normally black in color, but in the slide glass of the invention it is preferably a transparent film. The reason is that, as the slide glass of the invention is used in analysis of genes by measuring the intensity under irradiation of fluorescence on the slide glass surface, the optical transmittance is required.

The optical transmittance of the slide glass on which a surface treatment layer is formed in the invention is preferably 50% or more, more preferably 60% or more, at the wavelength of 400 nm to 500 nm (blue region).

Moreover, the transmittance is preferably 70% or more, more preferably 75% or more at the wavelength of 600 nm to 700 nm(red region). Depending on the optical transmittance in the respective region as described above, the intensity of the fluorescence label can be measured.

In producing diamond-like carbon having such optical transmittance, for example, by the ionizing vapor deposition process, one having higher transmittance can be produced with increase of the hydrogen gas content in a mixed gas of hydrocarbon gas and hydrogen gas.

The above-described ionizing vapor deposition process is a kind of DLC film formation processes devised by Weissmantel et al, in which an ion source comprises a cathode (thermal filament), an anodic grid as a counter electrode, and a metallic cylinder surrounding them.

The followings describe embodiment of carrying out the production of diamond-like carbon by which the optical transmittance is increased on the slide glass by the ionizing vapor deposition process.

For example, a diamond-like carbon (DLC) film of 10 nm in thickness was formed on a slide glass (Matsunaga Glass Co., S-1112) of 1 mm in thickness. The optical transmittance of the DLC film-coated slide glass was examined; the result was as follows. In the slide glass on which a DLC film was formed in an atmosphere containing no hydrogen gas at all (0% by volume), the optical transmittance was 88.7% at a wavelength of 400 nm. In the slide glass on which a DLC film was formed in an atmosphere containing 1% by volume of hydrogen gas, the optical transmittance was 89.4% at a wavelength of 400 nm. Moreover, in the slide glass on which a DLC film was formed in an atmosphere containing 80% by volume of hydrogen gas, the optical transmittance was 90.8% at a wavelength of 400 nm. As described above, it was found that the optical transmittance increases with increase of the ratio of hydrogen gas.

When the optical transmittance is measured at a wavelength of 700 nm, the slide glass on which a DLC film was formed in an atmosphere containing no hydrogen gas at all (0% by volume) showed 90.0% of the optical transmittance. The slide glass on which a DLC film was formed in an atmosphere containing 1% by volume of hydrogen gas showed 90.8% of the optical transmittance. The slide glass on which a DLC film was formed in an atmosphere containing 80% by volume of hydrogen gas showed 92% of the optical transmittance. As described above, it was found that the optical transmittance also increases with increase of the ratio of hydrogen gas at a wavelength of 700 nm similarly in the measurement at 400 nm.

On the other hand, the slide glass per se having no surface treatment layer (blank) exhibited 91.0% of the optical transmittance at a wavelength of 400 nm and 92% at 700 nm.

The optical transmittance was calculated from the following equation:

$$\text{Optical transmittance } T = (I/I_0) \times 100$$

I: Intensity of transmitted light in blank
$I_0$: Intensity of transmitted light when the surface treatment layer was formed

The reason why the presence of hydrogen gas enhances the transparency is considered that transparent sp3 orbital is contained in large quantities in the presence of hydrogen gas, while a large quantity of sp2 hybrid orbital is contained in the absence of hydrogen gas to cause coloring. The content of hydrogen gas, however, is preferably in 1 to 50% by volume because the presence of a large quantity of hydrogen gas retards the formation of diamond-like carbon film.

In the arc vapor disposition process, a direct current voltage is charged between a solid graphite material (cathodic vapor source) and a vacuum vessel (anode) to generate an arc discharge in vacuum, resulting in generation of carbon atom plasma in which the carbon ion is accelerated toward a substrate by charging a negative bias voltage to the substrate to form a film thereon.

In the laser vapor disposition process, for example, Nd:YAG (Yttrium Aluminum Garnet) laser (pulse oscillation) light is irradiated to a target plate of graphite, and the melted graphite is accumulated on a glass substrate to form a film.

Moreover, it is appropriate to form a reflection layer such as a metal evaporation film on the back of the slide glass on which a surface treatment layer has been formed.

The formation of such a reflection layer on the back of the slide glass is effective in enhancing the fluorescent reflection efficiency when the fluorescence is irradiated from the front surface, resulting in enhancing the sensitivity of the fluorescence analyzer. That is, the fluorescent spot can be observed clearly and a large number of spots can be analyzed at a time.

The reflection layer formed on the back of the slide glass may preferably be made as a single layer with Ti, Au, Pt, Nb, WC, and the like or as a multiple layer thereof. The thickness of the reflection layer may preferably be 100 nm or more, more preferably 1000 nm or more since it has to be coated evenly on the whole surface of the slide glass. The formation of such a reflection layer can be achieved generally using the vacuum evaporation method. Alternatively, another so far known technique suitable for forming the above-described reflection layer such as sputtering, an ion beam vapor deposition process, and the like, may properly be employed.

On the slide glass of the invention, a large number of biogenic materials such as genes, e.g. DNA probes, or proteins can be placed. Therefore, plural micro-sections may be provided on the slide glass surface, so that a number of oligonucleotide fragments can be kept on the one section. Such a slide glass may preferably be employed. In the respective micro-sections, a kind of DNA probes may properly be altered according to the purpose of use without any particular limitation.

There is no limitation as to the form of the slide glass, for example, it may be in a form of a plate such as film or sheet or of a disc. There is no particular limitation as to the thickness and size of the slide glass, and it may be made in the same range as those conventionally used.

Though the characteristics of the glass used as the slide glass substrate are not particularly limited, they may properly be chosen in consideration of various characters of the reactive material coated on the substrate surface, such as affinity.

The surface of the glass substrate may preferably be made rough intentionally in a range of 1 nm to 1000 nm Ra. Such rough surface is effective in increasing the surface area of the substrate and favorable in fixing a large quantity of DNA probe in high density.

The oligonucleotide fragments (probes) which can be fixed on the slide glass of the invention include single or double stranded DNA or RNA fragments, and there is no limitation on the number of bases. The immobilization of the oligonucleotide fragments may be achieved by formation of a chemical bond on the slide glass surface. For example, when the slide glass on which the surface treatment layer was formed with diamond-like carbon, the surface is activated, that is, it is processed for easy formation of the chemical bond with DNA, and can be bound to the amino group of the terminal DNA base.

The surface activation of the slide glass in this case is exemplified by the followings. The slide glass on a solid support was irradiated with ultraviolet ray in chlorine gas to chlorinate the surface of diamond-like carbon film, then irradiated with ultraviolet ray in ammonia gas to aminate, and then carboxylated with a suitable acid chloride or dicarboxylic acid anhydride; then the terminal carboxyl group is condensed with a carbodiimide or dicyclohexylcarbodiimide and N-hydroxysuccinimide under a dehydration condition. In this operation, it is possible to fix a group to which an active ester group such as N-hydroxysuccinimido ester is attached at the terminal of hydrocarbon group through an amide bond.

Thus, by activation of the slide glass surface, for example, tens of thousands of already elucidated DNA fragments (probes), proteins, peptides, and the like, can be kept thereon.

Additionally, it is also possible to bind an oligo dT primer on the slide glass, from which the objective cDNA is extended by reverse transcription reaction and bound to the slide glass at the same time.

Moreover, a large number of DNA strands are extended by PCR and bound on the slide glass.

After the DNA fragment was thus bound, a fluorescence-labeled DNA sample is poured thereon; the DNA sample hybridizes with a probe having the complementary sequence among the DNA fragments (probes) attached to the slide glass; thus, the DNA sequence of the sample can be elucidated as a fluorescent spot.

Particularly, in the slide glass for genetic analysis of the invention, since a reflection layer is formed on the back, the fluorescence spot can be observed clearly.

As described above, the slide glass of the invention allows much more definite analysis and characterization of the base sequence of a certain DNA than in the prior art according to the same manner as in the prior art. The slide glasses of the invention can be utilized in genetic analyses such as analyses of biogenic genomes, monitoring of gene expression, genomic mismatch, and the like, as well as in genetic diagnosis such as detection of mutation of oncogenes or development of drugs. The invention will be further explained by the following examples.

Example 1

The slide glass used in genetic analysis was provided as follows. A glass substrate of 25 mm (width) ×75 mm (length) ×1 mm (thickness) was used. On the surface of this glass substrate was formed a DLC film of 25 nm in thickness by the ionizing vapor deposition process using a mixed gas of 95% by volume of methane gas and 5% by volume of hydrogen gas to give a slide glass.

Then, the surface of this slide glass was chemically modified and activated.

That is, the surface of the glass substrate was chlorinated for 1 minute, then aminated for 10 minutes, and then immersed in an acid chloride for 10 minutes. This was then washed with ultrapure water-and immersed into an activation solution for direct activation. The composition of the activation solution comprises 1 mL of 1,4-dioxane, 25 mg of hydrogen cyanamide and 150 mg of N-hydroxysuccinimide, and the activation solution is prepared by dissolving them. The substrate was further washed with ultrapure water and dried at 65°C for activation.

On the surface of the slide glass prepared as described above, 2μL of FAMdA17 solution of 500pomol/mL concentration was dropped (spot). In this operation, ultrapure water or 1% formaldehyde was used as a buffer.

Then, the slide glass was incubated in a water/formamide (1/1) atmosphere at 65°C for 1 hour (drying).

For the resulting slide glass used for genetic analysis, the fluorescent intensity was measured. The fluorescent intensity was measured on an apparatus LAS-1000 Plus for 1 minute after spotting and after drying (65°C), and the result indicated the value obtained was better than in the slide used in the prior art.

In the slide glass of the invention, the fluorescent intensity after spotting and after drying was also better than that of the slide glass of the prior art after spotting and after drying. That is, in the slide glass of the invention, the spotted biogenic fragments such as DNAs or proteins were hold thereon without washing away, and the spot could be detected clearly.

On the other hand, in the slide glass of the prior art, the spotted biogenic fragments such as DNAs or proteins were washed away without kept on the slide glass, and the spot could not be detected clearly.

Example 2

The slide glass used in genetic analysis was provided as follows. A glass substrate of 25 mm (width) ×75 mm (length) ×1 mm (thickness) was used. A diamond film of 500 nm in thickness was then synthesized on the glass substrate. In the synthesis of the diamond film, a laser ablation process was used, wherein carbon was scattered from a target containing diamond in an atmosphere of oxygen gas and accumulated.

Subsequently, the surface of the slide glass was chemically modified and activated in the same manner as in Example 1. For the resulting slide glass used for genetic analysis, the fluorescent intensity was measured in the same manner as in Example 1. The fluorescent intensity of the slide glass of the invention after spotting and after drying was better than that of the prior art.

Example 3

The slide glass used in genetic analysis was provided as follows. A glass substrate of 25 mm (width) ×75 mm (length) ×1 mm (thickness) was used. A graphite film was formed on the glass substrate by the sputtering process. From the graphite-containing target, carbon was scattered and accumulated on the substrate in an argon atmosphere to form a graphite film of 25 μm in thickness thereon.

The surface of the slide glass was chemically modified and activated in the same manner as in Example 1. For the resulting slide glass used for genetic analysis, the fluorescent intensity was measured in the same manner as in Example 1. The fluorescent intensity of the slide glass of the invention after spotting and after drying was better than that of the prior art.

Industrial Applicability

The slide glasses of the invention allow much more definite analysis and identification of DNA base sequences than in the prior art even though an old apparatus or process is used in the invention.

The slide glasses of the invention can effectively be utilized in genetic analyses such as analyses of biogenic genomes, monitoring of gene expression, genomic mismatch, and the like, or in analyses of biogenic materials such as proteins.

Moreover, the slide glasses of the invention are useful in genetic diagnosis such as detection of mutation of oncogenes or development of drugs.

**Claims**

1. A slide glass in which a surface treatment layer is formed on the surface of a glass substrate.

2. A slide glass as claimed in Claim 1, wherein the surface treatment layer is diamond,-diamond-like carbon, or carbon-type material, or a mixture thereof, or a laminating layer thereof.

3. A slide glass as claimed in Claim 1 or 2, wherein the optical transmittance of the slide glass on which the above-described diamond-like carbon is formed is 50% or more at a wavelength of 400 nm to 500 nm.

4. A slide glass as claimed in Claim 1 or 2, wherein the optical transmittance of the slide glass on which the above-described diamond-like carbon is formed is 70% or more at a wavelength of 600 nm to 700 nm.

5. A slide glass as claimed in Claim 1 or 2, wherein the thickness of the above-described surface treatment layer is in 1 nm to 1000 nm.

6. A slide glass as claimed in any one of Claims 1 to 5, wherein the above-described diamond-like carbon is prepared by an ionizing vapor deposition process in a mixed gas containing 1-99 % by volume of hydrogen gas and residual 99-1 % by volume of methane gas.

7. A slide glass in which a reflection layer is formed on the back of a glass substrate.

8. A slide glass as claimed in any one of Claims 1 to 7, wherein an oligonucleotide fragment is kept on the above-described surface treatment layer.

9. A slide glass as claimed in any one of Claims 1 to 7, wherein a protein or peptide is kept on the above-described surface treatment layer.

10. A method for analyzing a biogenic material such as gene, protein, peptide, and the like using a slide glass as claimed in any one of Claims 1 to 9.

Fig. 1

DLC surface

Utilization of amino group
of A, G, C

$H_2N$ —

Amide bonding

## INTERNATIONAL SEARCH REPORT

| | International application No. |
|---|---|
| | PCT/JP00/06792 |

**A. CLASSIFICATION OF SUBJECT MATTER**
Int.Cl⁷ G01N33/552, 33/556, 33/48, C12N15/09

According to International Patent Classification (IPC) or to both national classification and IPC

**B. FIELDS SEARCHED**

Minimum documentation searched (classification system followed by classification symbols)
Int.Cl⁷ G01N33/552, 33/556, 33/48, C12N15/09

Documentation searched other than minimum documentation to the extent that such documents are included in the fields searched
Jitsuyo Shinan Koho 1922-1996 Toroku Jitsuyo Shinan Koho 1994-2000
Kokai Jitsuyo Shinan Koho 1971-2000 Jitsuyo Shinan Toroku Koho 1996-2000

Electronic data base consulted during the international search (name of data base and, where practicable, search terms used)
BIOSIS

**C. DOCUMENTS CONSIDERED TO BE RELEVANT**

| Category* | Citation of document, with indication, where appropriate, of the relevant passages | Relevant to claim No. |
|---|---|---|
| X | JP, 2000-63154, A (Mitsubishi Chemical Corporation), 29 February, 2000 (29.02.00) (Family: none) | 1,2,8-10 |
| A | JP, 8-240555, A (Kobe Steel, Ltd.), 17 September, 1996 (17.09.96) & US, 5777372, A | 1-10 |
| X | JP, 8-82747, A (Nikon Corporation), 26 March, 1996 (26.03.96) (Family: none) | 7 |
| X | JP, 9-292572, A (Nikon Corporation), 11 November, 1997 (11.11.97) (Family: none) | 7 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 October, 2000 (25.10.00) | 07 November, 2000 (07.11.00) |

| Name and mailing address of the ISA/ Japanese Patent Office | Authorized officer |
|---|---|
| Facsimile No. | Telephone No. |

Form PCT/ISA/210 (second sheet) (July 1992)